# EUROPEAN PATENT APPLICATION

(11) **EP 4 197 540 A1**
(43) Date of publication of application: **21.06.2023**
(21) Application number: 21855988.8
(22) Date of filing: 12.08.2021
(51) Int. Cl.: A61K 31/558, A61K 45/00, A61P 27/06

(54) **DRUG FORMULATION CONTAINING SEPETAPROST**

(30) Priority: 13.08.2020 JP 2020136796
(71) Applicant: Santen Pharmaceutical Co., Ltd., Kita-ku Osaka-shi Osaka 530-8552 (JP)
(72) Inventor: YAMAMOTO Yasuko, Ikoma-shi, Nara 630-0101 (JP); TANIGUCHI Takazumi, Ikoma-shi, Nara 630-0101 (JP); SHIMAZAKI Atsushi, Ikoma-shi, Nara 630-0101 (JP); KIMURA Erika, Ikoma-shi, Nara 630-0101 (JP)
(74) Representative: Müller-Boré & Partner Patentanwälte PartG mbB
(86) International application number: PCT/JP2021/029701
(87) International publication number: WO 2022/034909

(57) **Abstract**

The purpose of the present invention is to identify a drug formulation for the treatment or prevention of glaucoma or ocular hypertension, wherein the drug formulation exhibits an effect in patients with drug-refractory glaucoma or ocular hypertension. Sepetaprost is expected to exhibit an excellent intraocular pressure-lowering action in patients with otherwise drug-refractory glaucoma or ocular hypertension. The drug formulation according to the present invention containing sepetaprost as an active ingredient is therefore useful as a drug formulation that can treat or prevent glaucoma or ocular hypertension in patients with otherwise drug-refractory glaucoma or ocular hypertension.

## Description

### TECHNICAL FIELD

The present invention relates to a pharmaceutical formulation for treating or preventing glaucoma or ocular hypertension comprising sepetaprost as an active ingredient, the pharmaceutical formulation being administered to a patient for whom other therapeutic agent for glaucoma or ocular hypertension are inadequately effective.

### BACKGROUND ART

Glaucoma is an intractable ocular disease that can lead to blindness due to damage in internal tissues of the eye (such as retina, optic nerve) caused by an elevated intraocular pressure resulting from various pathogenesis. As methods for treating glaucoma, intraocular pressure lowering therapies are commonly performed, typically including drug therapy, laser therapy, and surgical therapy.

Drugs used for the drug therapy include sympathomimetic agents (non-selective stimulants such as dipivefrin, α₂ receptor agonists such as brimonidine), sympatholytic agents (β-receptor blockers such as timolol, befunolol, carteolol, nipradilol, betaxolol, levobunolol, metipranolol (Metipranolol), α₁ receptor blockers such as bunazosin hydrochloride), parasympathomimetic agents (such as pilocarpine), carbonic anhydrase inhibitors (such as acetazolamide), prostaglandins (such as isopropyl unoprostone, latanoprost, travoprost, bimatoprost), and Rho kinase inhibitors (ripasudil).

Among these drugs, eye drops containing latanoprost have been widely used worldwide since its international launch in 1996 because of its potent intraocular pressure lowering action and favorable tolerability. However, it is known that there are a certain number of patients for whom latanoprost is inadequately effective.

On the other hand, sepetaprost is a compound represented by formula (1) 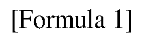 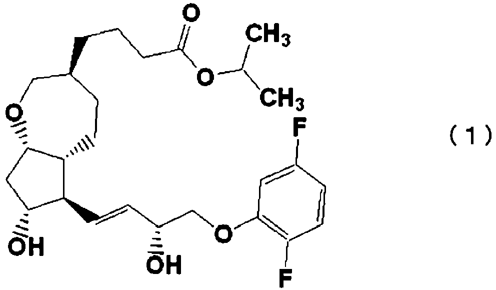 and is referred to as one of a vast number of compounds in Patent Document 1, which describes that these compounds have a potent and sustained intraocular pressure lowering action and could be a therapeutic agent for glaucoma.

However, none of the documents describe how sepetaprost is effective in a patient for whom other therapeutic agent for glaucoma or ocular hypertension is inadequately effective, and there are no other documents or studies reporting such effectiveness.

### PRIOR ART DOCUMENTS

### Patent Document

Patent Document 1: International Publication No. 2011/013651

### SUMMARY OF INVENTION

### Technical Problem

Identifying a pharmaceutical formulation for treating or preventing glaucoma or ocular hypertension that is effective in a patient for whom therapeutic agent for glaucoma or ocular hypertension is inadequately effective is a very interesting subject.

### Solution to Problem

Therefore, as a result of intensive studies, the present inventors have found that sepetaprost exhibits an excellent intraocular pressure lowering action in a patient for whom other therapeutic agents for glaucoma or ocular hypertension is inadequately effective, thus completing the present invention. Specifically, the present invention provides the following.
[1] A pharmaceutical formulation for treating or preventing glaucoma or ocular hypertension comprising sepetaprost as an active ingredient, the pharmaceutical formulation being administered to a patient for whom other therapeutic agent for glaucoma or ocular hypertension is inadequately effective.
[2] The pharmaceutical formulation according to the above-mentioned [1], wherein the treatment or prevention of glaucoma or ocular hypertension is treatment or prevention of glaucoma or ocular hypertension by further lowering an intraocular pressure with the active ingredient following treatment or prevention of glaucoma or ocular hypertension with the other therapeutic agent for glaucoma or ocular hypertension.
[3] The pharmaceutical formulation according to the above mentioned [1] or [2], wherein an active ingredient of the other therapeutic agent for glaucoma or ocular hypertension is prostaglandin F_{2α} derivative.
[4] The pharmaceutical formulation according to any one of the above-mentioned [1] to [3], wherein an active ingredient of the other therapeutic agent for glaucoma or ocular hypertension is latanoprost.
[5] The pharmaceutical formulation according to any one of the above-mentioned [1] to [4], wherein a content of sepetaprost is 0.0001% to 0.05% (w/v).
[6] The pharmaceutical formulation according to any one of the above-mentioned [1] to [5], wherein a content of sepetaprost is 0.001% to 0.005% (w/v).
[7] The pharmaceutical formulation according to any one of the above-mentioned [1] to [6], wherein a content of sepetaprost is 0.002% (w/v).
[8] The pharmaceutical formulation according to any one of the above-mentioned [1] to [7], wherein the pharmaceutical formulation is an eye drop.
[9] A pharmaceutical formulation for treating or preventing glaucoma or ocular hypertension comprising sepetaprost as an active ingredient, wherein the glaucoma is a glaucoma refractory to treatment of glaucoma with an active ingredient other than sepetaprost and the ocular hypertension is an ocular hypertension refractory to treatment of ocular hypertension with the other active ingredient.
[10] A method of treating or preventing glaucoma or ocular hypertension, comprising: administering a pharmaceutical formulation comprising sepetaprost as an active ingredient to a patient, wherein the patient is a patient for whom other therapeutic agent for glaucoma or ocular hypertension is inadequately effective.
[11] The method according to the above-mentioned [10], wherein the treatment or prevention of glaucoma or ocular hypertension is treatment or prevention of glaucoma or ocular hypertension by further lowering an intraocular pressure with the active ingredient following treatment or prevention of glaucoma or ocular hypertension with the other therapeutic agent for glaucoma or ocular hypertension.
[12] The method according to the above-mentioned [10] or [11], wherein an active ingredient of the other therapeutic agent for glaucoma or ocular hypertension is prostaglandin F_{2α} derivative.
[13] The method according to any one of the above-mentioned [10] to [12], wherein an active ingredient of the other therapeutic agent for glaucoma or ocular hypertension is latanoprost.
[14] The method according to any one of the above-mentioned [10] to [13], wherein a content of sepetaprost in the pharmaceutical formulation is 0.0001% to 0.05% (w/v).
[15] The method according to any one of the above-mentioned [10] to [14], wherein a content of sepetaprost in the pharmaceutical formulation is 0.001% to 0.005% (w/v).
[16] The method according to any one of the above-mentioned [10] to [15], wherein a content of sepetaprost in the pharmaceutical formulation is 0.002% (w/v).
[17] The method according to any one of the above-mentioned [10] to [16], wherein the administration is eye administration.
[18] A method of treating or preventing glaucoma or ocular hypertension, comprising: administering a pharmaceutical formulation comprising sepetaprost as an active ingredient to a patient, wherein the glaucoma is a glaucoma refractory to treatment of glaucoma with an active ingredient other than sepetaprost and the ocular hypertension is an ocular hypertension refractory to treatment of ocular hypertension with the other active ingredient.
[19] A method of treating or preventing glaucoma or ocular hypertension comprising administering a pharmaceutical formulation comprising sepetaprost as an active ingredient to a patient, the method comprising steps below:
   (1) a first treatment step of administering a therapeutic agent for glaucoma or ocular hypertension other than sepetaprost to a patient;
   (2) a step of determining whether or not the first treatment step has an inadequate effect of treatment or an inadequate effect of prevention;
   (3) a second treatment step of further administering a pharmaceutical formulation comprising sepetaprost as an active ingredient to the patient when the first treatment step has an inadequate effect of treatment or an inadequate effect of prevention.
[20] A use of the pharmaceutical formulation according to any one of the above-mentioned [1] to [9] in the manufacture of a medicament for treating or preventing glaucoma or ocular hypertension.
   The present invention further relates to the following.
[21] A pharmaceutical formulation for use in the treatment or prevention of glaucoma or ocular hypertension comprising sepetaprost as an active ingredient, the pharmaceutical formulation being administered to a patient for whom other therapeutic agent for glaucoma or ocular hypertension is inadequately effective.
[22] A pharmaceutical formulation for use in the treatment or prevention of glaucoma or ocular hypertension comprising sepetaprost as an active ingredient, wherein the glaucoma is a glaucoma refractory to treatment of glaucoma with an active ingredient other than sepetaprost and the ocular hypertension is an ocular hypertension refractory to treatment of ocular hypertension with the other active ingredient.
[23] A composition for treating or preventing glaucoma or ocular hypertension comprising sepetaprost as an active ingredient, the composition being administered to a patient for whom other therapeutic agent for glaucoma or ocular hypertension is inadequately effective.
[24] A composition for treating or preventing glaucoma or ocular hypertension comprising sepetaprost as an active ingredient, wherein the glaucoma is a glaucoma refractory to treatment of glaucoma with an active ingredient other than sepetaprost and the ocular hypertension is an ocular hypertension refractory to treatment of ocular hypertension with the other active ingredient.

Two or more of the components from each of the above-mentioned [1] to [24] can be arbitrarily selected and combined.

### Advantageous Effects of Invention

As will be described in detail in Examples later, sepetaprost was suggested to exhibit an excellent intraocular pressure lowering action in a patient for whom other therapeutic agent for glaucoma or ocular hypertension is inadequately effective. Therefore, sepetaprost is useful as a pharmaceutical formulation for treating or preventing glaucoma or ocular hypertension in a patient for whom other therapeutic agent for glaucoma or ocular hypertension is inadequately effective.

### DESCRIPTION OF EMBODIMENTS

Hereafter, the present invention is described in detail with reference to Embodiments.

### [Pharmaceutical formulation]

Sepetaprost contained in the pharmaceutical formulation of the present invention is a compound (CAS registration number: 1262873-06-2) represented by formula (1) below: 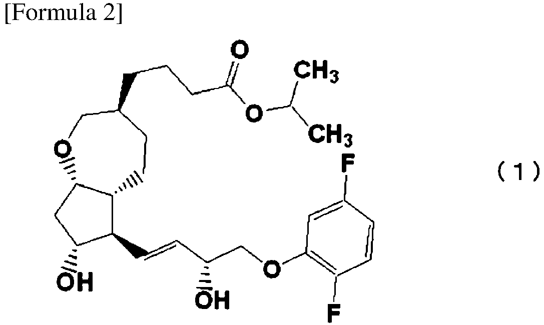 and also referred to as 2-propanyl 4-{(3S,5aR,6R,7R,8aS)-6-[(1E,3R)-4-(2,5-difluorophenoxy)-3-hydroxy-1-buten-1-yl]-7-hydroxyoctahydro-2H-cyclopenta[b]oxepin-3-yl}butanoate.

Sepetaprost can be produced in accordance with a method disclosed in International Publication No. 2011/013651 (Patent Document 1), common methods used in the art or the like.

When there are geometric isomers and/or optical isomers in sepetaprost, those isomers are also included in the scope of the present invention.

When there is proton tautomerism in sepetaprost, those tautomers (keto form and enol form) are also included in the present invention.

When there is crystal polymorphism and/or crystal polymorph group (crystal polymorph system) in sepetaprost, those crystal polymorphs and/or crystal polymorph group (crystal polymorph system) are also included in the present invention. Here, the crystal polymorph group (crystal polymorph system) refers to a crystal form at each stage and/or the whole of the crystal forms when the crystal form changes in various ways depending on the conditions and/or state (this state also includes a formulated state) of production, crystallization, preservation and the like of these crystals.

Sepetaprost may take a form of a hydrate or a solvate.

A content of sepetaprost comprised in the pharmaceutical formulation of the present invention is not particularly limited, which may vary depending on dosage forms, and a lower limit of the content thereof is, for example, 0.000001% (w/v), preferably 0.000005% (w/v), 0.00001% (w/v), 0.00005% (w/v), more preferably 0.0001% (w/v), still more preferably 0.0005% (w/v), and particularly preferably 0.001% (w/v). An upper limit of the above-mentioned content is, for example, 5% (w/v), preferably 3% (w/v), 1% (w/v), 0.5% (w/v), 0.1% (w/v), more preferably 0.05% (w/v), still more preferably 0.01% (w/v), and particularly preferably 0.005% (w/v). In more detail, the above-mentioned content may be in ranges of a combination of any of the above-mentioned lower limit and upper limit, and for example, it is 0.000001 to 3% (w/v), preferably 0.000005 to 1% (w/v), 0.00001 to 0.5% (w/v), 0.00005 to 0.1% (w/v), more preferably 0.0001 to 0.05% (w/v), still more preferably 0.0005 to 0.01% (w/v), particularly preferably 0.001 to 0.005% (w/v). More specifically, 0.001% (w/v), 0.002% (w/v), 0.003% (w/v), 0.004% (w/v), 0.005% (w/v) and ranges with these contents as an upper limit and a lower limit are preferable, and 0.002% (w/v) is the most preferable. The above-mentioned contents may be given as a preferred content, for example, when the pharmaceutical formulation is an eye drop. Here, "% (w/v)" means a mass (g) of an active ingredient (sepetaprost) or an additive (such as surfactant) contained in 100 mL of the pharmaceutical formulation. For example, 0.01% (w/v) sepetaprost means that the content of sepetaprost contained in 100 mL of the pharmaceutical formulation is 0.01 g.

When sepetaprost is in the form of a hydrate or a solvate, the content of sepetaprost may be calculated based on any of a free form, a hydrate or a solvate of sepetaprost.

### [Additive]

An additive may be used in the pharmaceutical formulation of the present invention as necessary. Examples of the additive that may be added include a surfactant, a buffer, an isotonic agent, a stabilizer, a preservative, antioxidant, a thickening agent, a base, and a pH adjuster. Each additive may be used individually or two or more additives may be used in combination as appropriate, and an appropriate amount may be blended.

When the pharmaceutical formulation of the present invention is an eye drop, it is preferable that water is a base.

For a pH of the pharmaceutical formulation of the present invention, 4.0 to 8.0 is preferred and 5.0 to 7.0 is more preferred.

### [Usage]

As will be shown in the pharmacological tests section below, sepetaprost exhibited an adequate intraocular pressure lowering action in monkeys with an inadequate response to latanoprost. Therefore, the pharmaceutical formulation of the present invention is expected to exhibit an excellent intraocular pressure lowering action in a patient with an inadequate response to other therapeutic agent for glaucoma or ocular hypertension or in a patient for whom other therapeutic agent for glaucoma or ocular hypertension is inadequately effective, and is useful as an agent for treating or preventing glaucoma, and/or an agent for treating or preventing ocular hypertension, and/or an intraocular pressure lowering agent. Examples of glaucoma in the present invention include primary open-angle glaucoma, secondary open-angle glaucoma, normal tension glaucoma, hypersecretion glaucoma, primary angle closure glaucoma, secondary angle closure glaucoma, plateau iris glaucoma, combined-mechanism glaucoma, developmental glaucoma, steroid induced glaucoma, exfoliation glaucoma, amyloid glaucoma, neovascular glaucoma, malignant glaucoma, capsular glaucoma of the lens and plateau iris syndrome, preferably primary open-angle glaucoma, normal tension glaucoma and primary angle closure glaucoma, and particularly, the pharmaceutical formulation of the present invention is effective for primary open-angle glaucoma. In addition, glaucoma and ocular hypertension in the scope of the treatment or prevention in the present invention may be glaucoma refractory to treatment of glaucoma with an active ingredient other than sepetaprost and ocular hypertension refractory to treatment of ocular hypertension with an active ingredient other than sepetaprost respectively.

It is preferable that the pharmaceutical formulation of the present invention is administered to a patient for whom other therapeutic agent for glaucoma or ocular hypertension is inadequately effective. The other therapeutic agent for glaucoma or ocular hypertension refers to any of the therapeutic agents for glaucoma or ocular hypertension that contain an active ingredient other than sepetaprost (other active ingredient). The active ingredient other than sepetaprost specifically includes non-selective sympathomimetic agents, α₂ receptor agonists, α₁ receptor blockers, β receptor blockers, parasympathomimetic agents, carbonic anhydrase inhibitors, prostaglandins and Rho kinase inhibitors.

Specific examples of the non-selective sympathomimetic agents include dipivefrin, specific examples of the α₂ receptor agonists include brimonidine and apraclonidine, specific examples of the α₁ receptor blockers include bunazosin, specific examples of the β receptor blockers include timolol, befunolol, carteolol, nipradilol, betaxolol, levobunolol and metipranolol, specific examples of the parasympathomimetic agents include pilocarpine, specific examples of the carbonic anhydrase inhibitors include dorzolamide, brinzolamide and acetazolamide, specific examples of the prostaglandins include latanoprost, isopropyl unoprostone, bimatoprost and travoprost, and specific examples of the Rho kinase inhibitors include fasudil and ripasudil. As the active ingredient of the other therapeutic agent for glaucoma or ocular hypertension in the present invention, prostaglandins are preferred, prostaglandin F_{2α} derivative is more preferred, latanoprost is still more preferred, latanoprost eye drop is particularly preferred, and 0.005% latanoprost eye drop is most preferred.

A patient for whom other therapeutic agent for glaucoma or ocular hypertension is inadequately effective refers to a patient who does not obtain an adequate effect from treatment with the other therapeutic agent for glaucoma or ocular hypertension, and can also be referred to as a patient with an inadequate response (refractory) to other therapeutic agent for glaucoma or ocular hypertension. Therefore, the above-mentioned agent for treating or preventing glaucoma, and/or the agent for treating or preventing ocular hypertension, and/or the intraocular pressure lowering agent can be said to be an agent for treating or preventing glaucoma, and/or an agent for treating or preventing ocular hypertension, and/or an intraocular pressure lowering agent in a patient with an inadequate response (refractory) to the other therapeutic agent for glaucoma or ocular hypertension. The patient for whom the other therapeutic agent for glaucoma or ocular hypertension is inadequately effective includes a patient whose intraocular pressure is not lowered adequately by receiving administration of the other therapeutic agent for glaucoma or ocular hypertension. Specifically, in the case of human patients, a patient who has a lowering rate of the post-treatment intraocular pressure relative to the pre-treatment intraocular pressure by the treatment with the other therapeutic agent for glaucoma or ocular hypertension (intraocular pressure lowering rate: {[pre-treatment intraocular pressure (mmHg) - post-treatment intraocular pressure (mmHg)] / [pre-treatment intraocular pressure (mmHg)]} × 100%) being, for example, 18% or less, preferably 17% or less, more preferably 16% or less, still more preferably 15% or less, even more preferably 14% or less, further more preferably 13% or less, particularly preferably 12% or less, and most preferably 10% or less is included. The intraocular pressure lowering rate mentioned here is an example and the lowering rate can vary depending on a mechanism of action of the other therapeutic agent for glaucoma or ocular hypertension. For example, a patient for whom the effect of treatment with the other therapeutic agent for glaucoma or ocular hypertension is less than 50% of the intraocular pressure lowering rate that could be essentially achieved is also included in the patients for whom the other therapeutic agent for glaucoma or ocular hypertension is inadequately effective in the present invention. In addition, in the case of human patients, a patient who has a lowering width of the post-treatment intraocular pressure relative to the pre-treatment intraocular pressure by the treatment with the other therapeutic agent for glaucoma or ocular hypertension (= intraocular pressure lowering width: [pre-treatment intraocular pressure (mmHg) - post-treatment intraocular pressure (mmHg)]) being, for example, 4.5 mmHg or less, preferably 4.2 mmHg or less, more preferably 4 mmHg or less, still more preferably 3.7 mmHg or less, even more preferably 3.5 mmHg or less, further more preferably 3.2 mmHg or less, particularly preferably 3 mmHg or less, and most preferably 2.5 mmHg or less is included. The intraocular pressure lowering width mentioned here is an example and the lowering width can vary depending on a mechanism of action of the other therapeutic agent for glaucoma or ocular hypertension. For example, a patient for whom the effect of treatment with the other therapeutic agent for glaucoma or ocular hypertension is less than 50% of the intraocular pressure lowering width that could be essentially achieved is also included in the patients for whom the other therapeutic agent for glaucoma or ocular hypertension is inadequately effective in the present invention. The treatment with the other therapeutic agent for glaucoma or ocular hypertension is usually administration to an eye 1 to 3 times a day, 1 to 3 drops per dose, for a period of 1 week or more, preferably 2 weeks or more, more preferably 4 weeks or more, still more preferably 2 months or more, particularly preferably 6 months or more, and most preferably 1 year or more. The patient for whom the other therapeutic agent for glaucoma or ocular hypertension is inadequately effective includes a patient who cannot use or be treated with the other therapeutic agent for glaucoma or ocular hypertension due to adverse effects or other reasons. Patients targeted by the pharmaceutical formulation of the present invention are mammals, including domestic animals such as cows and pigs, rabbits, monkeys, dogs, cats, and humans, preferably humans.

The pharmaceutical formulation of the present invention can further lower the intraocular pressure in a patient treated with the other therapeutic agent for glaucoma or ocular hypertension who has a level of the intraocular pressure lowering rate and intraocular pressure lowering width that may be considered inadequately effective as described above. It is preferred that the lowering rate of the post-treatment intraocular pressure relative to the pre-treatment intraocular pressure (intraocular pressure lowering rate) by the pharmaceutical formulation of the present invention is, for example, in the case of human patients, at least 5%, for example 6% or more, preferably 7% or more, more preferably 8% or more, still more preferably 9% or more, even more preferably 10% or more, further more preferably 11% or more, particularly preferably 12% or more, and most preferably 13% or more. An upper limit of the intraocular pressure lowering rate by the pharmaceutical formulation of the present invention is, for example, in the case of human patients, 40% or less, preferably 35% or less, more preferably 30% or less, still more preferably 28% or less, even more preferably 26% or less, further more preferably 24% or less, particularly preferably 22% or less, and most preferably 20% or less, and any combination of the lower limit and the upper limit mentioned-above can be selected as appropriate. A preferred range of the intraocular pressure lowering rate by the pharmaceutical formulation of the present invention is, for example, in the case of human patients, 5 to 40%, preferably 7 to 35%, and more preferably 9 to 30%. In addition, it is preferred that lowering width of the post-treatment intraocular pressure relative to the pre-treatment intraocular pressure (intraocular pressure lowering width) by the pharmaceutical formulation of the present invention is, for example, in the case of human patients, at least 1.0 mmHg or more, preferably 1.2 mmHg or more, more preferably 1.4 mmHg or more, still more preferably 1.6 mmHg or more, even more preferably 1.8 mmHg or more, further more preferably 2.0 mmHg or more, particularly preferably 2.5 mmHg or more, and most preferably 2.9 mmHg or more. An upper limit of the intraocular pressure lowering width by the pharmaceutical formulation of the present invention is, for example, in the case of human patients, 10.0 mmHg or less, preferably 8.0 mmHg or less, more preferably 6.0 mmHg or less, still more preferably 5.5 mmHg or less, even more preferably 5.0 mmHg or less, further more preferably 4.5 mmHg or less, particularly preferably 4.0 mmHg or less and most preferably 3.2 mmHg or less, and any combination of the widths of the lower limit and the upper limit mentioned-above can be selected as appropriate. A preferred range of the intraocular pressure lowering width by the pharmaceutical formulation of the present invention is, for example, in the case of human patients, 1.0 to 10.0 mmHg, preferably 1.4 to 8.0 mmHg, and more preferably 1.8 to 6.0 mmHg. The "lowering rate of the post-treatment intraocular pressure relative to the pre-treatment intraocular pressure (intraocular pressure lowering rate) by the pharmaceutical formulation of the present invention" and the "lowering width (intraocular pressure lowering width)" defined here refer to an intraocular pressure lowering rate and an intraocular pressure lowering width in a second treatment step to be shown in (3) of [Method for administration] later, and they do not include an intraocular pressure lowering rate and an intraocular pressure lowering width by the treatment with the other therapeutic agent for glaucoma or ocular hypertension as shown in a first treatment step to be shown in (1) of [Method for administration]. Therefore, the pharmaceutical formulation of the present invention comprising sepetaprost as an active ingredient can lower intraocular pressure in addition to the treatment effect of the other therapeutic agent for glaucoma or ocular hypertension comprising latanoprost or other substance as an active ingredient.

The pharmaceutical formulation of the present invention may be used in combination (e.g., as a kit) with 1 or more, preferably 1 to 3, more preferably 1 or 2 additional therapeutic agents for glaucoma or ocular hypertension and may contain additional active ingredients. There are no restrictions on the additional therapeutic agents for glaucoma or ocular hypertension. Specifically, the therapeutic agents for glaucoma or ocular hypertension that are commercially available or in development are preferred, and the commercially available therapeutic agents for glaucoma or ocular hypertension are more preferred. More specifically, examples thereof include the therapeutic agents for glaucoma or ocular hypertension containing non-selective sympathomimetic agents, α₂ receptor agonists, α₁ receptor blockers, β-receptor blockers, parasympathomimetic agents, carbonic anhydrase inhibitors, prostaglandins, Rho kinase inhibitors, and the like as an active ingredient. There are no restrictions on additional active ingredients. Specifically examples thereof include non-selective sympathomimetic agents, α₂ receptor agonists, α₁ receptor blockers, β-receptor blockers, parasympathomimetic agents, carbonic anhydrase inhibitors, prostaglandins, and Rho kinase inhibitors. When the pharmaceutical formulation of the present invention is used in combination with the additional therapeutic agent for glaucoma or ocular hypertension, or contains the additional active ingredient, the "lowering rate of the post-treatment intraocular pressure relative to the pre-treatment intraocular pressure (intraocular pressure lowering rate) by the pharmaceutical formulation of the present invention " and the "lowering width (intraocular pressure lowering width)" described above refer to the portion excluding the effect of the additional therapeutic agent for glaucoma or ocular hypertension or the additional active ingredient.

Specific examples of the non-selective sympathomimetic agents include dipivefrin, specific examples of the α₂ receptor agonists include brimonidine and apraclonidine, specific examples of the α₁ receptor blockers include bunazosin, specific examples of the β receptor blockers include timolol, befunolol, carteolol, nipradilol, betaxolol, levobunolol and metipranolol, specific examples of the parasympathomimetic agents include pilocarpine, specific examples of the carbonic anhydrase inhibitors include dorzolamide, brinzolamide and acetazolamide, specific examples of the prostaglandins include isopropyl unoprostone, bimatoprost and travoprost, and specific examples of the Rho kinase inhibitors include fasudil and ripasudil.

### [Dosage form]

The pharmaceutical formulation of the present invention can be administered orally or parenterally, e.g., by eye administration, intravitreal administration, conjunctival sac administration, anterior chamber administration, subconjunctival administration, sub-Tenon's administration or lacrimal point plug administration. Dosage forms of the pharmaceutical formulation of the present invention include eye drops, ophthalmic ointments, injections, lacrimal point plugs, tablets, capsules, granules and powders, and eye drops are particularly preferred.

### [Dosage and administration]

The dosage and administration of the pharmaceutical formulation of the present invention is not restricted provided that the dosage and administration are adequate to achieve the desired drug efficacy, and can be selected based on symptoms of the disease, the age and weight of a patient, the dosage form of the pharmaceutical formulation and the like as appropriate.

Specifically, in the case of eye drops, a dose of 1 to 5 drops, preferably 1 to 3 drops, more preferably 1 to 2 drops, particularly preferably 1 drop, can be administered to an eye, 1 to 4 times a day, preferably 1 to 3 times a day, more preferably 1 to 2 times a day, particularly preferably once a day, at a frequency of daily to weekly. It is preferable to administer a drop to an eye once a day. Here, one drop is typically about 0.01 to about 0.1 mL, preferably about 0.015 to about 0.07 mL, and more preferably, about 0.02 to about 0.05 mL.

### [Method for administration]

The pharmaceutical formulation of the present invention can be administered to a patient for whom the above-mentioned other therapeutic agent for glaucoma or ocular hypertension is inadequately effective. That is, the method for administration of the pharmaceutical formulation of the present invention comprises administering a pharmaceutical formulation comprising sepetaprost as an active ingredient to a patient, and the patient is a patient for whom the other therapeutic agents for glaucoma or ocular hypertension are inadequately effective. This allows for the treatment or prevention of glaucoma or ocular hypertension of the patient. As described above, it may well be said that the pharmaceutical formulation of the present invention can be administered to a patient with an inadequate response (refractory) to the above-mentioned other therapeutic agent for glaucoma or ocular hypertension.

Also, according to the method for administration of the pharmaceutical formulation of the present invention, treatment or prevention of glaucoma or ocular hypertension with other active ingredient may be further performed in advance. This allows for the treatment or prevention of glaucoma or ocular hypertension by treating or preventing glaucoma or ocular hypertension with the other therapeutic agent for glaucoma or ocular hypertension and then further lowering intraocular pressure with sepetaprost (active ingredient).

According to the method for administration of the pharmaceutical formulation of the present invention, it may be administered orally or parenterally, e.g., by eye administration, intravitreal administration, conjunctival sac administration, anterior chamber administration, subconjunctival administration, sub-Tenon's administration or lacrimal point plug administration, but eye administration is preferred.

In addition, when treatment or prevention with the above-mentioned other therapeutic agent for glaucoma or ocular hypertension is not adequately effective or is not expected to be adequately effective, the pharmaceutical formulation of the present invention can also be administered subsequently. More specifically, the method for administration of the pharmaceutical formulation of the present invention comprises steps below:
(1) a first treatment step comprising treating or preventing glaucoma or ocular hypertension by the above-mentioned active ingredient other than sepetaprost;
(2) an optional step of determining whether or not the first treatment step has an inadequate effect of treatment or an inadequate effect of prevention;
(3) a second treatment step comprising administering a pharmaceutical formulation comprising sepetaprost as an active ingredient to treat or prevent glaucoma or ocular hypertension following the first treatment step.

By administering the pharmaceutical formulation of the present invention to a patient according to the method for administration described above, regardless of an effect of the treatment or prevention of the above-mentioned other active ingredient, or when the effect is inadequate, the pharmaceutical formulation of the present invention can additionally provide an intraocular pressure lowering action and the like and further treat or prevent glaucoma or ocular hypertension.

The above-mentioned method for administration will be more specifically described below. First, the above-mentioned other therapeutic agent for glaucoma or ocular hypertension (the other active ingredient) than sepetaprost is administered to treat or prevent glaucoma or ocular hypertension. Then, in the case that the treatment effect of the other active ingredient is expected to be inadequate or the prevention effect of the other active ingredient is expected to be inadequate, it is judged to be "a glaucoma refractory to treatment of glaucoma with the other active ingredient" or "an ocular hypertension refractory to treatment of a ocular hypertension with the other active ingredient," and the pharmaceutical formulation of the present invention comprising sepetaprost as an active ingredient is subsequently administered. Here, examples of the case that "the treatment effect is inadequate" and "the prevention effect is expected to be inadequate" include the case in which the intraocular pressure lowering rate and intraocular pressure lowering width are at the level of the "patient who does not obtain an adequate effect from treatment by the other therapeutic agent for glaucoma or ocular hypertension " as described above, and also the case in which the absolute value of the intraocular pressure is still high enough to be considered glaucoma or ocular hypertension, for example, 22 mmHg or more, preferably 21.5 mmHg or more, more preferably 21 mmHg or more, still more preferably 20.5 mmHg or more, and particularly preferably 20 mmHg or more. When curing or the like of glaucoma or ocular hypertension remains unattained by the administration of the above-mentioned other active ingredient, or when there remains a potential risk of recurrence, it is appropriate to use the pharmaceutical formulation of the present invention as a means of second-line administration. Thus, the pharmaceutical formulation of the present invention can further advance and complete the treatment or prevention of glaucoma or ocular hypertension in the case that the treatment and the like with the above-mentioned other active ingredient such as latanoprost had been inadequate, and also in the case that the treatment or prevention was thought to have been adequately completed with the other active ingredient. This is an unexpected action and effect, and provides a solution to a problem different from conventional ones.

For example, if latanoprost is given as the other active ingredient, when further reduction of intraocular pressure is required after the treatment or prevention of glaucoma or ocular hypertension is performed with latanoprost, or when re-elevation of intraocular pressure (rebound) occurs or is expected to occur after the treatment or prevention with latanoprost, the pharmaceutical formulation of the present invention is expected to be effective in lowering the intraocular pressure or preventing the re-elevation of intraocular pressure (rebound).

The description in detail of the pharmaceutical formulation and the like of the present invention mentioned above also applies to aspects of the present invention to be described below.

One aspect of the present invention is a method of treating or preventing glaucoma or ocular hypertension comprising: administering a pharmaceutical formulation comprising sepetaprost as an active ingredient to a patient, wherein the patient is a patient for whom other therapeutic agent for glaucoma or ocular hypertension is inadequately effective.

One aspect of the present invention is a method of treating or preventing glaucoma or ocular hypertension, comprising: administering a pharmaceutical formulation comprising sepetaprost as an active ingredient to a patient, wherein the glaucoma is a glaucoma refractory to treatment of glaucoma with an active ingredient other than sepetaprost and the ocular hypertension is an ocular hypertension refractory to treatment of ocular hypertension with the other active ingredient.

One aspect of the present invention is a method of treating or preventing glaucoma or ocular hypertension comprising: administering a pharmaceutical formulation comprising sepetaprost as an active ingredient to a patient, the method comprising steps below: (1) a first treatment step of administering a therapeutic agent for glaucoma or ocular hypertension other than sepetaprost to a patient; (2) a step of determining whether or not the first treatment step has an inadequate effect of treatment or an inadequate effect of prevention; (3) a second treatment step of further administering a pharmaceutical formulation comprising sepetaprost as an active ingredient to the patient when the first treatment step has an inadequate effect of treatment or an inadequate effect of prevention.

One aspect of the present invention is a use of a pharmaceutical formulation comprising sepetaprost as an active ingredient in the manufacture of a medicament for treating or preventing glaucoma or ocular hypertension, wherein the pharmaceutical formulation is administered to a patient for whom other therapeutic agent for glaucoma or ocular hypertension is inadequately effective.

One aspect of the present invention is a use of a pharmaceutical formulation comprising sepetaprost as an active ingredient in the manufacture of a medicament for treating or preventing glaucoma or ocular hypertension, wherein the glaucoma is a glaucoma refractory to treatment of glaucoma with active ingredient other than sepetaprost and the ocular hypertension is an ocular hypertension refractory to treatment of ocular hypertension with the other active ingredient.

One aspect of the present invention is a pharmaceutical formulation for use in the treatment or prevention of glaucoma or ocular hypertension comprising sepetaprost as an active ingredient, the pharmaceutical formulation being administered to a patient for whom other therapeutic agent for glaucoma or ocular hypertension is inadequately effective.

One aspect of the present invention is a pharmaceutical formulation for use in the treatment or prevention of glaucoma or ocular hypertension comprising sepetaprost as an active ingredient, wherein the glaucoma is a glaucoma refractory to treatment of glaucoma with an active ingredient other than sepetaprost and the ocular hypertension is an ocular hypertension refractory to treatment of ocular hypertension with the other active ingredient.

One aspect of the present invention is a composition for treating or preventing glaucoma or ocular hypertension comprising sepetaprost as an active ingredient, the composition being administered to a patient for whom other therapeutic agent for glaucoma or ocular hypertension is inadequately effective.

One aspect of the present invention is a composition for treating or preventing glaucoma or ocular hypertension comprising sepetaprost as an active ingredient, wherein the glaucoma is a glaucoma refractory to treatment of glaucoma with an active ingredient other than sepetaprost and the ocular hypertension is an ocular hypertension refractory to treatment of ocular hypertension with the other active ingredient.

Hereafter, Examples with reference to pharmacological tests of the present invention are shown. These Examples are for the purpose of better understanding of the present invention and are not intended to limit the scope of the present invention.

### EXAMPLES

### [Pharmacological tests]

### [Example 1]

### 1. Selection of monkeys with inadequate response to latanoprost

Latanoprost eye drop was administered to cynomolgus monkeys (monkeys with normal intraocular pressure) and each individual animal was examined for responsiveness to latanoprost to select monkeys with an inadequate response to latanoprost.

### <Test materials>

(Eye drop)
   Latanoprost eye drop: Xalatan(R) Eye Drops 0.005% (w/v) (Pfizer Japan Inc.)
(Test animals)
   Cynomolgus monkeys: 32 animals (Sex/ male)

### <Methods for administration and Measurement>

(1) An intraocular pressure was measured immediately before administering the test eye drop and was defined as the pre-administration intraocular pressure value (0 hour).
(2) The test eye drop was administered once (20 µL/eye) to one eye (right eye) of the test animals using a PIPETMAN. The contralateral eye was untreated.
(3) An intraocular pressure was measured at 2, 4, and 6 hours after the administration. The intraocular pressure was measured three times for each to calculate its average value. An intraocular pressure change value (mmHg) was calculated as the difference from the pre-administration intraocular pressure value (0 hour) at each measurement time point.

The test was conducted as a crossover study. A physiological saline solution was administered as a test eye drop to a control group. Two series of administering the latanoprost eye drop were conducted and designated as a latanoprost (first time) group and a latanoprost (second time) group, respectively.

### <Selection of monkeys with inadequate response to latanoprost>

From the cynomolgus monkeys tested (32 animals), those individuals who had an intraocular pressure lowering width of less than 1 mmHg at all time points of 2, 4, and 6 hours after the administration of latanoprost (first time) and latanoprost (second time) were selected as monkeys with an inadequate response to latanoprost (7 animals).

### <Results>

Table 1 shows the intraocular pressure change values (mmHg) of the selected monkeys with an inadequate response to latanoprost (7 animals) for each test eye drop treatment group at time points of 2, 4, and 6 hours after the administration.

**[Table 1]**

| Group | Hours after administration | | |
|---|---|---|---|
| | 2 | 4 | 6 |
| Control | -0.6 | -0.1 | -0.5 |
| Latanoprost (first time) | 1.0 | -0.2 | -0.6 |
| Latanoprost (second time) | 0.4 | 0.0 | -0.1 |

Table 1 reveals that little change over time in the intraocular pressure was observed in the latanoprost (first time) group and the latanoprost (second time) group of the selected monkeys with an inadequate response to latanoprost (7 animals) up to 6 hours after the administration, in the same manner as in the control group.

### [Example 2]

### 2. Test for effect of latanoprost on lowering intraocular pressure in monkeys with inadequate response to latanoprost

Latanoprost eye drop was administered to the monkeys with an inadequate response to latanoprost, and its effect on lowering the intraocular pressure at 10 and 12 hours after the administration was evaluated.

### <Test materials>

(Eye drop)
   Latanoprost eye drop: Xalatan(R) Eye Drops 0.005% (w/v) (Pfizer Japan Inc.)
(Test animals)
   Cynomolgus monkeys: 7 animals (sex: male) selected in "1. Selection of monkeys with inadequate response to latanoprost"

### <Methods for administration and Measurement>

(1) An intraocular pressure was measured immediately before administering the test eye drop and was defined as the pre-administration intraocular pressure value (0 hour).
(2) The test eye drop was administered once (20 µL/eye) to one eye (right eye) of the test animals using a PIPETMAN. The contralateral eye was untreated.
(3) An intraocular pressure was measured at 10 and 12 hours after the administration. The intraocular pressure was measured three times for each to calculate its average value. An intraocular pressure change value (mmHg) was calculated as the difference from the pre-administration intraocular pressure value (0 hour) at each measurement time point.

### <Results>

Table 2 shows the intraocular pressure change values (mmHg) of the tested monkeys with an inadequate response to latanoprost at time points of 10 and 12 hours after the administration of the latanoprost eye drop.

**[Table 2]**

| Group | Hours after administration | |
|---|---|---|
| | 10 | 12 |
| Latanoprost | -0.5 | -1.2 |

When latanoprost eye drop was administered to the monkeys with an inadequate response to latanoprost, as revealed by Table 2, the intraocular pressure lowering effect was inadequate as the intraocular pressure lowering width was as small as 1.2 mmHg even 12 hours after the administration.

### [Example 3]

### 3. Test for effect of sepetaprost on lowering intraocular pressure in monkeys with inadequate response to latanoprost

Sepetaprost eye drop was administered to monkeys with an inadequate response to latanoprost, and its effect on lowering the intraocular pressure at 10 and 12 hours after the administration was evaluated.

### <Test materials>

(Eye drop)
   Sepetaprost eye drop: Sepetaprost was dissolved in a base, and then, a sepetaprost eye drop with a concentration of 0.002% (w/v) was prepared by using a commonly used method.
(Test animals)
   Cynomolgus monkeys: 7 animals (sex: male) selected in "1. Selection of monkeys with inadequate response to latanoprost"

### <Methods for administration and Measurement>

(1) An intraocular pressure was measured immediately before administering the test eye drop and was defined as the pre-administration intraocular pressure value (0 hour).
(2) The test eye drop was administered once (20 µL/eye) to one eye (right eye) of the test animals using a PIPETMAN. The contralateral eye was untreated.
(3) An intraocular pressure was measured at 10 and 12 hours after the administration. The intraocular pressure was measured three times for each to calculate its average value. An intraocular pressure change value (mmHg) was calculated as the difference from the pre-administration intraocular pressure value (0 hour) at each measurement time point.

The test was conducted as a crossover study. The above-mentioned base was administered as a test eye drop to a control group. Two series of administering the sepetaprost eye drop were conducted and designated as a sepetaprost (first time) group and a sepetaprost (second time) group, respectively.

### <Results>

Table 3 shows the intraocular pressure change value (mmHg) of the tested monkeys with an inadequate response to latanoprost at time points of 10 and 12 hours after the administration by each test eye drop treatment group.

**[Table 3]**

| Group | Hours after administration | |
|---|---|---|
| | 10 | 12 |
| Control | -0.7 | -1.0 |
| Sepetaprost (first time) | -3.1 | -3.3 |
| Sepetaprost (second time) | -2.6 | -3.4 |

When the sepetaprost eye drop was administered to the monkeys with an inadequate response to latanoprost, as revealed by Table 3, the intraocular pressure lowering action was significant as the intraocular pressure lowering width at time points of 10 and 12 hours after the administration was 3.1 and 3.3 mmHg for the first time group and 2.6 and 3.4 mmHg for the second time group respectively. The results demonstrated that an adequate intraocular pressure lowering effect can be obtained by administering sepetaprost in the monkeys with an inadequate response to latanoprost.

From these results, sepetaprost has been expected to exhibit an excellent intraocular pressure lowering action in a patient with an inadequate response to other therapeutic agent for glaucoma or ocular hypertension and has been suggested to exhibit an excellent intraocular pressure lowering effect in a patient for whom the other therapeutic agent for glaucoma or ocular hypertension is inadequately effective. This has indicated that sepetaprost can exert an effect in the treatment or prevention of glaucoma or ocular hypertension in a patient for whom the other therapeutic agent for glaucoma or ocular hypertension is inadequately effective.

### INDUSTRIAL APPLICABILITY

Sepetaprost is expected to exhibit an excellent intraocular pressure lowering action in a patient with an inadequate response to the other therapeutic agent for glaucoma or ocular hypertension. Therefore, the pharmaceutical formulation of the present invention comprising sepetaprost as an active ingredient is useful as a pharmaceutical formulation which can treat or prevent glaucoma or ocular hypertension in a patient for whom the other therapeutic agent for glaucoma or ocular hypertension is inadequately effective.

## Claims

1. A pharmaceutical formulation for treating or preventing glaucoma or ocular hypertension comprising sepetaprost as an active ingredient, the pharmaceutical formulation being administered to a patient for whom other therapeutic agent for glaucoma or ocular hypertension is inadequately effective.

2. The pharmaceutical formulation according to Claim 1, wherein the treatment or prevention of glaucoma or ocular hypertension is treatment or prevention of glaucoma or ocular hypertension by further lowering an intraocular pressure with the active ingredient following treatment or prevention of glaucoma or ocular hypertension with the other therapeutic agent for glaucoma or ocular hypertension.

3. The pharmaceutical formulation according to Claim 1 or 2, wherein an active ingredient of the other therapeutic agent for glaucoma or ocular hypertension is prostaglandin F_{2α} derivative.

4. The pharmaceutical formulation according to any one of Claims 1 to 3, wherein an active ingredient of the other therapeutic agent for glaucoma or ocular hypertension is latanoprost.

5. The pharmaceutical formulation according to any one of Claims 1 to 4, wherein a content of sepetaprost is 0.0001% to 0.05% (w/v).

6. The pharmaceutical formulation according to any one of Claims 1 to 5, wherein a content of sepetaprost is 0.001% to 0.005% (w/v).

7. The pharmaceutical formulation according to any one of Claims 1 to 6, wherein a content of sepetaprost is 0.002% (w/v).

8. The pharmaceutical formulation according to any one of Claims 1 to 7, wherein the pharmaceutical formulation is an eye drop.

9. A pharmaceutical formulation for treating or preventing glaucoma or ocular hypertension comprising sepetaprost as an active ingredient, wherein the glaucoma is a glaucoma refractory to treatment of glaucoma with an active ingredient other than sepetaprost and the ocular hypertension is an ocular hypertension refractory to treatment of ocular hypertension with the other active ingredient.

10. A method of treating or preventing glaucoma or ocular hypertension, comprising: administering a pharmaceutical formulation comprising sepetaprost as an active ingredient to a patient, wherein the patient is a patient for whom other therapeutic agent for glaucoma or ocular hypertension is inadequately effective.

11. The method according to Claim 10, wherein the treatment or prevention of glaucoma or ocular hypertension is treatment or prevention of glaucoma or ocular hypertension by further lowering an intraocular pressure with the active ingredient following treatment or prevention of glaucoma or ocular hypertension with the other therapeutic agent for glaucoma or ocular hypertension.

12. The method according to Claim 10 or 11, wherein an active ingredient of the other therapeutic agent for glaucoma or ocular hypertension is prostaglandin F_{2α} derivative.

13. The method according to any one of Claims 10 to 12, wherein an active ingredient of the other therapeutic agent for glaucoma or ocular hypertension is latanoprost.

14. The method according to any one of Claims 10 to 13, wherein a content of sepetaprost in the pharmaceutical formulation is 0.0001% to 0.05% (w/v).

15. The method according to any one of Claims 10 to 14, wherein a content of sepetaprost in the pharmaceutical formulation is 0.001% to 0.005% (w/v).

16. The method according to any one of Claims 10 to 15, wherein a content of sepetaprost in the pharmaceutical formulation is 0.002% (w/v).

17. The method according to any one of Claims 10 to 16, wherein the administration is eye administration.

18. A method of treating or preventing glaucoma or ocular hypertension, comprising: administering a pharmaceutical formulation comprising sepetaprost as an active ingredient to a patient, wherein the glaucoma is a glaucoma refractory to treatment of glaucoma with an active ingredient other than sepetaprost and the ocular hypertension is an ocular hypertension refractory to treatment of ocular hypertension with the other active ingredient.

19. A method of treating or preventing glaucoma or ocular hypertension comprising administering a pharmaceutical formulation comprising sepetaprost as an active ingredient to a patient, the method comprising steps below:
(1) a first treatment step of administering a therapeutic agent for glaucoma or ocular hypertension other than sepetaprost to a patient;
(2) a step of determining whether or not the first treatment step has an inadequate effect of treatment or an inadequate effect of prevention;
(3) a second treatment step of further administering a pharmaceutical formulation comprising sepetaprost as an active ingredient to the patient when the first treatment step has an inadequate effect of treatment or an inadequate effect of prevention.

20. A use of the pharmaceutical formulation according to any one of Claims 1 to 9, in the manufacture of a medicament for treating or preventing glaucoma or ocular hypertension.
